# EUROPÄISCHE PATENTSCHRIFT

(11) **EP 0 721 478 B1**
(45) Veröffentlichungstag und Bekanntmachung des Hinweises auf die Patenterteilung: **26.11.2003**
(21) Anmeldenummer: 94927632.3
(22) Anmeldetag: 20.09.1994
(51) Int. Cl.: C08G 63/685, C08G 69/26, C08G 69/44

(54) **PYRROLIDONGRUPPENHALTIGE POLYESTER UND POLYAMIDE**
PYRROLIDONE-GROUP-CONTAINING POLYESTERS AND POLYAMIDES
POLYESTERS ET POLYAMIDES CONTENANT DES GROUPES PYRROLIDONE

(30) Priorität: 30.09.1993 DE 4333238
(43) Veröffentlichungstag der Anmeldung: 17.07.1996
(73) Patentinhaber: BASF AKTIENGESELLSCHAFT, 67056 Ludwigshafen (DE)
(72) Erfinder: NGUYEN KIM, Son, D-69502 Hemsbach (DE); BREITENBACH, Jörg, D-53545 Linz (DE); SANNER, Axel, D-67227 Frankenthal (DE); HÖSSEL, Peter, D-67105 Schifferstadt (DE); LANG, Siegfried, D-67071 Ludwigshafen (DE)
(86) Internationale Anmeldenummer: EP9403141
(87) Internationale Veröffentlichungsnummer: WO95009194

(56) Entgegenhaltungen:
- GB-A- 1 483 457
- US-A- 2 993 021
- US-A- 4 418 189

## Beschreibung

Die vorliegende Erfindung betrifft neue pyrrolidongruppenhaltige Polyurethane und Polyharnstoffe, Verfahren zu ihrer Herstellung sowie die Verwendung solcher pyrrolidongruppenhaltiger Polyester und Polyamide auf speziellen technischen Gebieten.

Polyvinylpyrrolidon (PVP) und radikalisch erzeugte Copolymerisate des Vinylpyrrolidons, z.B. mit Vinylacetat, finden bislang in verschiedenen technischen Bereichen Anwendung. Dabei werden insbesondere ihre Eigenschaften als Komplexbildner, Filmbildner, Depotbildner, Enzymträger, Binder- und Klebrohstoff ausgenutzt. Die Anwendungen liegen auf dem kosmetischen und pharmazeutischen Sektor und im Nahrungsmittel-, Textil-, Waschmittel- und nicht zuletzt im Polymer-Bereich, etwa als Solubilisator oder Schutzkolloid.

PVP und seine Copolymerisate lassen jedoch eine Reihe von Mängeln bei diesen Anwendungen erkennen, insbesondere ihre unzureichende biologische Abbaubarkeit oder Eliminierbarkeit, ihr Toxizitätspotential (das monomere Vinylpyrrolidon wurde im Tierversuch als cancerogen eingestuft) und ihre teilweise Unverträglichkeit mit bestimmten Sacchariden (beispielsweise in Mischungen von PVP mit Maltodextrinen). Auch die anwendungstechnischen Eigenschaften des PVP und seiner Copolymerisate sind vielfach noch verbesserungsbedürftig.

Aus der US-A 2 993 021 (1) sind Polyester bekannt, die durch Polykondensation von Bispyrrolidonderivaten der Struktur 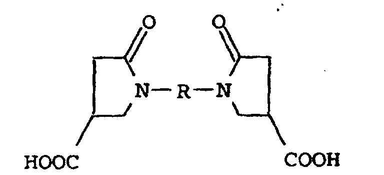 in der R eine Alkylen-, Oxalkylen-, Thialkylen- oder Azalkylen-Brücke mit 2 bis 12 C-Atomen bezeichnet, z.B. 1,2-Ethylen oder 1,3-Propylen, mit Polyhydroxyverbindung, z.B. Ethylenglykol oder Propylenglykol, erhalten werden. Diese Polyester eignen sich unter Umständen als Weichmacher für Kunststoffe und zur Herstellung von Alkyd-Harzen, welche bekanntermaßen als Grundstoffe in der Lackindustrie eine bedeutende Rolle spielen.

In der Literaturstelle Israel Journal of Chemistry, Bd. 10, 1972, S. 949-957 (2) werden hochtemperaturbeständige Polyamide beschrieben, die durch Reaktion von aromatischen Diaminen wie Benzidin, 4,4'-Diaminodiphenylsulfon, 4,4'-Diaminodiphenylether oder p-Phenylendiamin mit Bispyrrolidondicarbonsäuren, erhalten durch Umsetzung von Itaconsäure mit besagten aromatischen Diaminen, hergestellt werden. Diese Polyamide sind offenbar schwerlöslich, da sie sich nur in konzentrierter Schwefelsäure und einige von ihnen teilweise im Dimethylformamid oder Dimethylsulfoxid lösen. Als Verwendung wird der Einsatz in Klebstofformulierungen nahegelegt.

Aufgabe der vorliegenden Erfindung war es, als Ersatz für PVP und seine Copolymerisate neue Mittel für die genannten Einsatzgebiete bereitzustellen, die die Nachteile des Standes der Technik nicht mehr aufweisen.

Gegenstand der vorliegenden Erfindung sind pyrrolidongruppenhaltige Polyurethane oder Polyharnstoffe und pyrrolidongruppenhaltige Polycarboxylate, welche durch Umsetzung von pyrrolidongruppenhaltigen Polyestern oder Polyamiden der allgemeinen Formel V bis VII 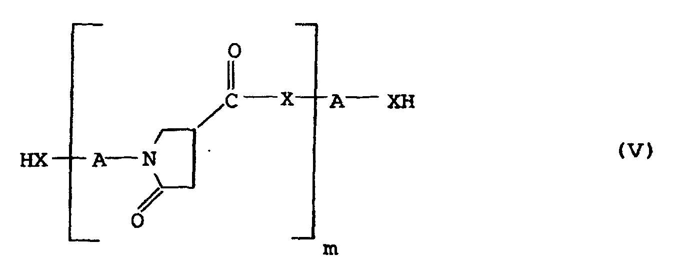 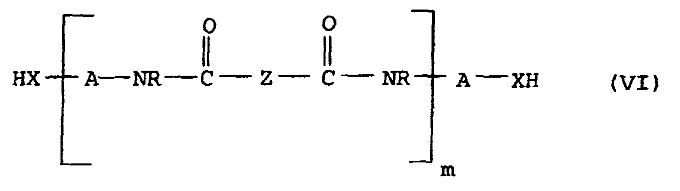 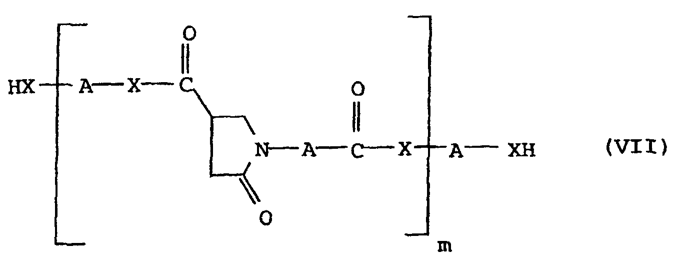 in denen
- R: für Wasserstoff oder C₁- bis C₄-Alkyl steht, wobei zwei Reste R unter Ausbildung eines Sechsringes miteinander verbunden sein können,
- X: Sauerstoff oder die Gruppe -NR- bezeichnet,
- A: C₁- bis C₂₀-Alkylen, welches durch ein oder mehrere nicht benachbarte Sauerstoffatome, Schwefelatome oder funktionelle Gruppen -NR-, wobei das N-Atom protoniert oder quaternisiert vorliegen kann, -CO-, -CO-O-, -CO-NR-, -SO- oder -SO₂- unterbrochen sein und zusätzliche funktionelle Gruppen -COOH oder - SO₃H tragen kann, C₆- bis C₂₀-Cycloalkylen, C₆- bis C₂₀-Arylen, welches durch Sauerstoff, Schwefel oder eine Gruppe -NR-, -SO- oder -SO₂- unterbrochen sein und zusätzliche funktionelle Gruppen -COOH oder -SO₃H tragen kann, oder eine Mischung solcher Gruppen bedeutet,
- Z: eine Gruppe der Formel VIII 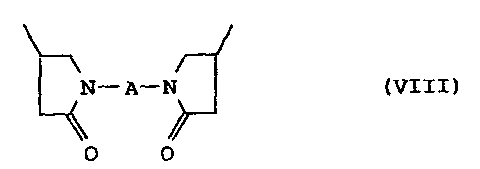 oder eine Mischung von Gruppen der Formel VIII mit Gruppe A bezeichnet und
- m: für eine Zahl von 1 bis 50 steht,
mit Diisocyanaten der allgemeinen Formel OCN-D-NCO, in der D für C₂- bis C₈-Alkylen, C₅- bis C₁₀-Cycloalkylen, Phenylen oder C₁- bis C₄-Alkylphenylen steht, bzw. Dianhydriden der allgemeinen Formel 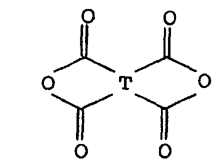 in der T für einen tetravalenten Rest eines C₂- bis C₆-Alkans, eines C₅- bis C₁₀-Cycloalkans, von Benzol oder Naphthalin steht, erhältlich sind.

C₁- bis C₄-Alkyl für R bedeutet Ethyl, n-Propyl, iso-Propyl, n-Butyl, iso-Butyl, sec.-Butyl, tert.-Butyl oder insbesondere Methyl. Sind zwei Gruppen -NR- durch eine C₁- bis C₃-Alkylengruppe miteinander verbunden, können die beiden Reste R unter Ausbildung eines gesättigten Sechsringes mit zwei N-Atomen, insbesondere eines Piperazin-Ringes, auch miteinander verbunden sein.

Treten jeweils mehrere Brückenglieder A in den allgemeinen Formeln V bis VII auf, können diese gleich oder verschieden sein.

Die Umsetzung von Itaconsäure oder ihren Derivaten mit primäre Aminogruppen enthaltenden monomeren Verbindungen zu Pyrrolidonstrukturen ist prinzipiell bekannt. Man arbeitet vorteilhafterweise hierbei unter einer Inertgasatmosphäre, z. B. unter Stickstoff, in einem Lösungsmittel, vorzugsweise Wasser, und bei Temperaturen von etwa 90 bis 120°C.

Zur Herstellung der Verbindungen V setzt man zweckmäßigerweise Itaconsäure, Itaconsäureanhydrid, Itaconsäureester oder Itaconsäurehalogenide mit einem geringen Überschuß an Aminoalkoholen oder Diaminen der allgemeinen Formel HX-A-NH₂ um oder gibt anschließend als Überschuß eine geringe bis molare Menge eines anderen Aminoalkohols oder Diamins HX-A-NH₂ oder eines Diols der allgemeinen Formel HO-A-OH hinzu und läßt reagieren und polykondensiert gewünschtenteils bei höherer Temperatur bis zum Polykondensationsgrad m, so daß sich an beiden Enden der Polykondensatkette freie Amino- oder Hydroxylgruppe befinden, die für die Umsetzung mit Diisocyanat oder Dianhydrid befähigt sind.

Zur Herstellung der Verbindungen VI setzt man zweckmäßigerweise Itaconsäure, Itaconsäureanhydrid, Itaconsäureester oder Itaconsäurehalogenide mit Aminoalkoholen oder Diaminen der allgemeinen Formel HX-A-NH₂ im molaren Verhältnis von etwa 2:1 um, läßt danach mit einer geringen bis molaren Menge des gleichen oder eines anderen Aminoalkohols oder Diamins HX-A-NH₂ oder eines Diols der allgemeinen Formel HO-A-OH reagieren und polykondensiert gewünschtenfalls mit den gleichen oder anderen Aminoalkoholen oder Diaminen HX-A-NH₂ bei höherer Temperatur bis zum Polykondensationsgrad m, so daß sich an beiden Enden der Polykondensatkette freie Amino- oder Hydroxylgruppen befinden, die für die Umsetzung mit Diisocyanat oder Dianhydrid befähigt sind. Ein Teil der Bispyrrolidondicarbonsäure-Bausteine läßt sich durch Dicarbonsäuren der allgemeinen Formel HOOC-A-COOH ersetzen.

Zur Herstellung der Verbindungen VII setzt man zweckmäßigerweise Itaconsäure, Itaconsäureanhydrid, Itaconsäureester oder Itaconsäurehalogenide mit Aminocarbonsäuren der allgemeinen Formel H₂N-A-CO-Y, in der der Rest Y für OH, O-Alkyl, z. B. O-C₁- bis C₄-Alkyl, oder Halogen, z. B. Cl oder Br, steht, im molaren Verhältnis von etwa 1:1 um, läßt danach mit einer geringen bis molaren Menge eines Aminoalkohols oder Diamins der allgemeinen Formel HX-A-NH₂ oder eines Diols der allgemeinen Formel HO-A-OH reagieren und polykondensiert gewünschtenfalls mit dem gleichen oder mit anderen Aminoalkoholen oder Diaminen HX-A-NH₂ bei höherer Temperatur bis zum Polykondensationsgrad m, so daß sich an beiden Enden der Polykondensatkette freie Amino- oder Hydroxylgruppen befinden, die für die Umsetzung mit Diisocyanat oder Dianhydrid befähigt sind.

Als Diisocyanate OCN-D-NCO kommen C₂- bis C₈-Alkylendiisocyanate, z. B. 1,2-Ethylendiisocyanat, 1,4-Butylendiisocyanat, Hexamethylendiisocyanat oder Octamethylendiisocyanat, C₅- bis C₁₀-Cycloalkylendiisocyanate, z. B. 1,3-Cyclopentylendiisocyanat, 1,3- oder 1,4-Cyclohexylendiisocyanat oder Isophorondiisocyanat, o-, m- oder p-Phenylendiisocyanat oder (C₁- bis C₄-Alkyl)phenylendiisocyanate, z. B. Toluylendiisocyanat, in Betracht.

Der tetravalente Rest T in den verwendeten Dianhydriden steht beispielsweise für 1,1,2,2-substituiertes Ethan, 1,2,3,4-substituiertes Butan, 1,2,3,4- oder 1,2,4,5-substituiertes Cyclohexan, 1,2,3,4- oder 1,2,4,5-substituiertes Benzol oder 1,2,3,4- oder 2,3,6,7-substituiertes Naphthalin.

Die Umsetzung zu den erfindungsgemäßen pyrrolidongruppenhaltigen Polyurethanen oder Polyharnstoffen und zu den erfindungsgemäßen pyrrolidongruppenhaltigen Polycarboxylaten erfolgt nach den hierzu bekannten Methoden und bedarf deshalb keiner weiteren Erläuterung.

Die erfindungsgemäßen pyrrolidongruppenhaltigen Polyurethane oder Polyharnstoffe und die erfindungsgemäßen pyrrolidongruppenhaltigen Polycarboxylate eignen sich als Filmbildner und Konditioner in haarkosmetischen Zubereitungen.

Die beschriebenen Polykondensate sind leicht herstellbar, man benötigt beispielsweise keine organischen Lösungsmittel hierzu. Die Ausgangsverbindung Itaconsäure ist leicht und preiswert mittels fermentativer Prozesse aus Zuckern oder Melasse zugänglich.

Bei vielen Anwendungen sind die beschriebenen Polykondensate dem PVP und seinen Copolymerisaten überlegen.

### Herstellungsbeispiele

Allgemeine Herstellungsvorschrift A

Aus Itaconsäure und einem Aminoalkohol oder Diamin oder dem Gemisch aus diesen Reaktanten wird in bekannter Weise in Wasser als Lösungsmittel bei ca. 100°C unter Stickstoffatmosphäre ein Pyrrolidonderivat hergestellt. Anschließend wird das Wasser praktisch vollständig abdestilliert und durch Erhitzen auf ca. 250°C bei Normaldruck unter Stickstoffatmosphäre die Polykondensation durchgeführt. Sich in Form von Wasserdampf bildendes Kondensationswasser wird fortlaufend mittels Durchleiten eines Stickstoffstromes entfernt. Nach ca. 6 bis 15 Stunden ist die Umsetzung beendet. Die Verwendung eines Katalysators wie Orthophosphorsäure oder Natriumdihydrogenphosphat in der hierbei üblichen Menge verringert die Reaktionszeit auf ca. 3 bis 10 Stunden.

### Allgemeine Herstellungsvorschrift B

Aus Itaconsäure und Ethylendiamin im molaren Verhältnis von 2:1 wird in bekannter Weise in Wasser als Lösungsmittel bei ca. 100°C unter Stickstoffatmosphäre die Bispyrrolidondicarbonsäure der Formel XI 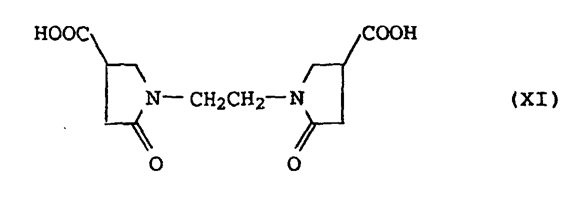 hergestellt. Anschließend wird das Wasser praktisch vollständig abdestilliert, das in Tabelle 1 angegebene Diamin oder Gemisch aus Diaminen sowie gegebenenfalls dort angegebene Dicarbonsäuren oder Aminocarbonsäuren zugegeben und durch Erhitzen auf ca. 250°C bei Normaldruck unter Stickstoffatmosphäre die Polykondensation durchgeführt. Sich in Form von Wasserdampf, bildendes Kondensationswasser wird fortlaufend mittels Durchleiten eines Stickstoffstromes entfernt. Nach ca. 6 bis 15 Stunden ist die Umsetzung beendet. Die Verwendung eines Katalysators wie Orthophosphorsäure oder Natriumdihydrogenphosphat in der hierbei üblichen Menge verringert die Reaktionszeit auf ca. 3 bis 10 Stunden.

### Herstellung eines pyrrolidongruppenhaltigen Polyharnstoffes

160 g (0,1 mol) eines mit einem leichten Überschuß Ethylendiamin hergestellten Poly(itaconsäure-ethylendiamins) wurden in 375 g einer Mischung aus Ethanol und Wasser im Vol.-Verhältnis 4:1 bei 20°C unter Rühren gelöst. Danach wurden 15,14 g (0,09 mol) Hexamethylendiisocyanat zugetropft. Die Innentemperatur überstieg dabei nicht 30°C. Anschließend wurde die Reaktionslösung noch 2 Stunden bei 20°C nachgerührt. Das Lösungsmittel wurde im Vakuumtrockenschrank bei 70°C entfernt. Man erhielt nach dem Trocknen einen wasserlöslichen harten Feststoff. Anstelle des Trocknens konnte man nach Zugabe weiteren Wassers durch Abdestillieren des Ethanols im schwachen Vakuum bei ca. 60°C eine wäßrige Lösung des Produktes erhalten.

## Patentansprüche

1. Pyrrolidongruppenhaltige Polyurethane oder Polyharnstoffe und pyrrolidongruppenhaltige Polycarboxylate, erhältlich durch Umsetzung von pyrrolidongruppenhaltigen Polyestern oder Polyamiden der allgemeinen Formel V bis VII 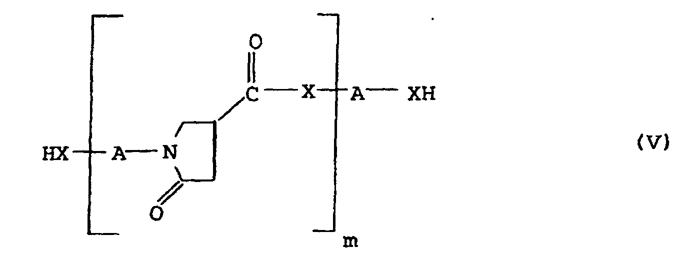 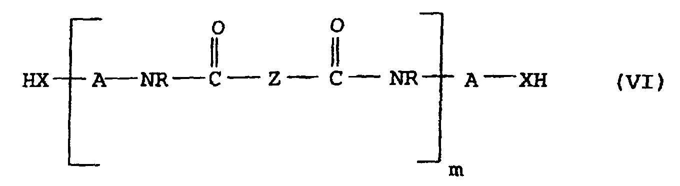 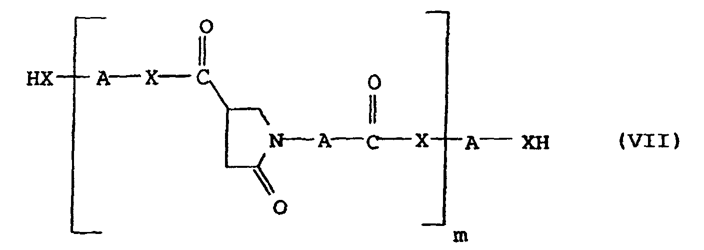 in denen
R für Wasserstoff oder C₁- bis C₄-Alkyl steht, wobei zwei Reste R unter Ausbildung eines Sechsringes miteinander verbunden sein können,
X Sauerstoff oder die Gruppe -NR- bezeichnet,
A C₁- bis C₂₀-Alkylen, welches durch ein oder mehrere nicht benachbarte Sauerstoffatome, Schwefelatome oder funktionelle Gruppen -NR-, wobei das N-Atom protoniert oder quaternisiert vorliegen kann, -CO-, -CO-O-, -CO-NR-, -SO- oder -SO₂- unterbrochen sein und zusätzliche funktionelle Gruppen -COOH oder -SO₃H tragen kann, C₆- bis C₂₀-Cycloalkylen, C₆- bis C₂₀-Arylen, welches durch Sauerstoff, Schwefel oder eine Gruppe -NR-, -SO- oder -SO₂- unterbrochen sein und zusätzliche funktionelle Gruppen -COOH oder - SO₃H tragen kann, oder eine Mischung solcher Gruppen bedeutet,
Z eine Gruppe der Formel VIII 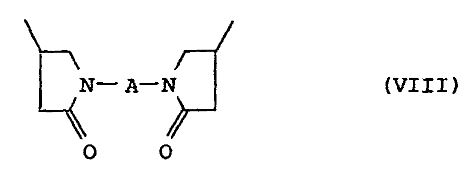 oder eine Mischung von Gruppen der Formel VIII mit Gruppen A bezeichnet und
m für eine Zahl von 1 bis 50 steht,
mit Diisocyanaten der allgemeinen Formel OCN-D-NCO, in der D für C₂- bis C₈-Alkylen, C₅- bis C₁₀-Cycloalkylen, Phenylen oder C₁- bis C₄-Alkylphenylen steht, bzw. Dianhydriden der allgemeinen Formel 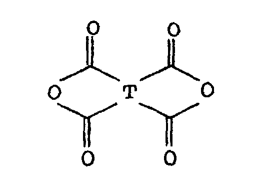 in der T für einen tetravalenten Rest eines C₂- bis C₆-Alkans, eines C₅- bis C₁₀-Cycloalkans, von Benzol oder Naphthalin steht.

2. Verwendung von pyrrolidongruppenhaltigen Polyurethanen und Polyharnstoffen und Polycarboxylaten gemäß Anspruch 1 als Filmbildner und Konditioner in haarkosmetischen Zubereitungen.

## Claims

1. A pyrrolidonyl-containing polyurethane or polyurea or a pyrrolidonyl-containing polycarboxylate obtainable by reacting a pyrrolidonyl-containing polyester or polyamide of the formulae V to VII 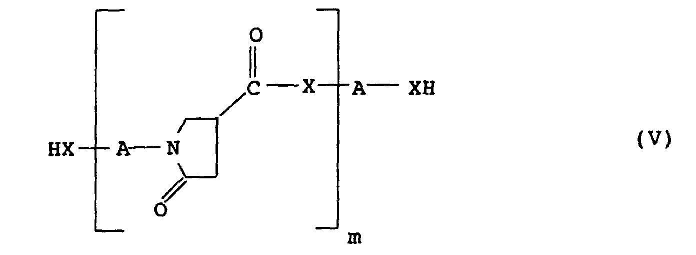 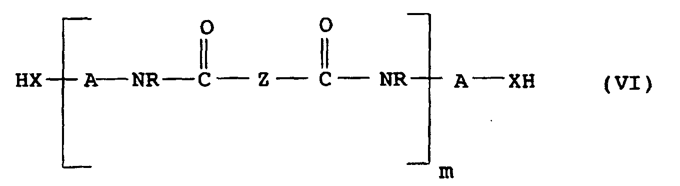 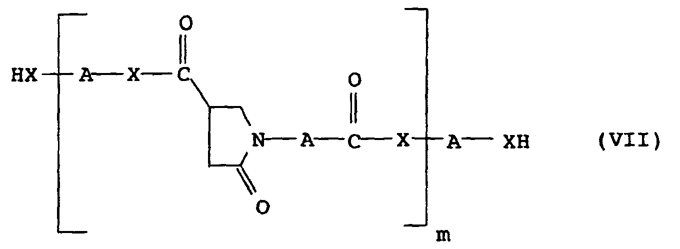 where
R is hydrogen or C₁- to C₄-alkyl, where two radicals R can be linked to one another to form a six-membered ring,
X is oxygen or -NR-,
A is C₁- to C₂₀-alkylene, which can be interrupted by one or more non-adjacent oxygen atoms, sulfur atoms or functional groups -NR-, where the nitrogen atom can be protonated or quaternized, -CO-, -CO-O-, -CO-NR-, -SO- or - SO₂-, and which may carry additional functional groups - COOH or -SO₃H, C₆- to C₂₀-cycloalkylene, C₆- to C₂₀-arylene, which may be interrupted by oxygen, sulfur or -NR-, -SO- or -SO₂- and may additionally carry functional groups -COOH or -SO₃H, or a mixture of such groups,
Z is a group of the formula VIII 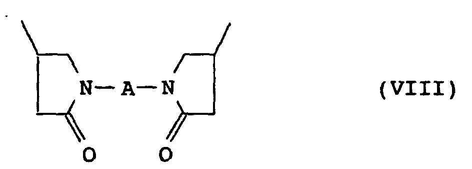 or a mixture of groups of formula VIII with group A and
m is a number from 1 to 50,
with diisocyanates of the formula OCN-D-NCO, where D is C₂- to C₈-alkylene, C₅- to C₁₀-cycloalkylene, phenylene or C₁- to C₄-alkylphenylene, or dianhydrides of the formula 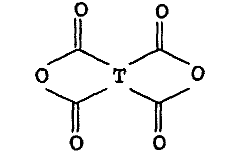 where T is a tetravalent radical of a C₂- to C₆-alkane, of a C₅- to C₁₀-cycloalkane, of benzene or of naphthalene.

2. The use of a pyrrolidonyl-containing polyurethane or polyurea or polycarboxylate as claimed in claim 1 as film-forming agents and conditioners in hair cosmetic formulations.

## Revendications

1. Polyuréthannes ou polyurées contenant des groupes pyrrolidone et polycarboxylates contenant des groupes pyrrolidone, pouvant être obtenus par réaction de polyesters ou de polyamides contenant des groupes pyrrolidone de formule générale V à VII 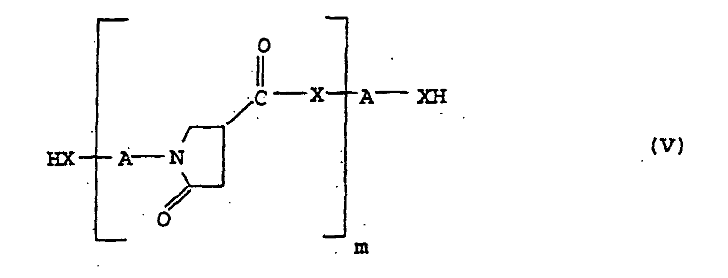 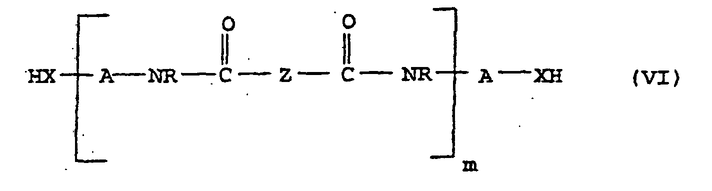 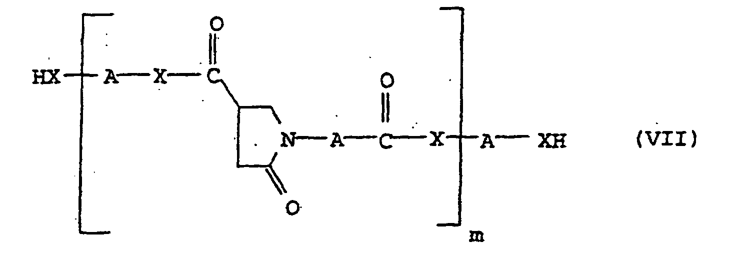 dans lesquelles
R désigne l'hydrogène ou un alkyle en C₁ à C₄, tandis que deux restes R peuvent être reliés entre eux par formation d'un cycle à six chaînons,
X désigne l'oxygène ou le groupe -NR-,
A représente un alkylène en C₁ à C₂₀, qui peut être interrompu par un ou plusieurs atomes d'oxygène, atomes de soufre ou groupes fonctionnels -NR- non vicinaux, tandis que l'atome N peut être protoné ou quaternisé, -CO-, -CO-O-, -CO-NR-, -SO- ou -SO₂-, et peut en outre porter des groupes fonctionnels - COOH ou -SO₃H additionnels, cycloalkylène en C₆ à C₂₀, arylène en C₆ à C₂₀, qui peut être interrompu par de l'oxygène, du soufre ou un groupe -NR-, -SO- ou -SO₂- et peut en outre porter des groupes fonctionnels -COOH ou -SO₃H additionnels, ou un mélange de tels groupes,
Z désigne un groupe de formule VIII 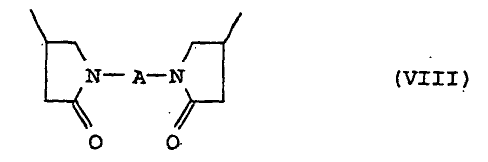 ou un mélange de groupes de formule VIII avec des groupes A, et
m représente un nombre de 1 à 50,
avec des düsocyanates de formule générale OCN-D-NCO, dans laquelle D représente un alkylène en C₂ à C₈, un cycloalkylène en C₅ à C₁₀, un phénylène ou un alkyl(C₁ à C₄)phénylène, ou des dianhydrides de formule générale 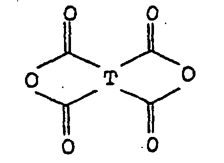 dans laquelle T représente un reste tétravalent d'un alcane en C₂ à C₆, d'un cycloalcane en C₅ à C₁₀, de benzène ou de naphtalène.

2. Utilisation de polyuréthannes et de polyurées et de polycarboxylates contenant des groupes pyrrolidone selon la revendication 1 comme agents filmogènes et conditionneurs dans des compositions cosmétiques capillaires.
